# EUROPÄISCHE PATENTSCHRIFT

(11) **EP 1 609 643 B1**
(45) Veröffentlichungstag und Bekanntmachung des Hinweises auf die Patenterteilung: **03.03.2010**
(21) Anmeldenummer: 05011917.1
(22) Anmeldetag: 02.06.2005
(51) Int. Cl.: B60H 3/00

(54) **Luftverbesserungsvorrichtung zur Verwendung in Kraftfahrzeugen und Kartusche für die Luftverbesserungsvorrichtung**
Air improving apparatus for use in motor vehicles and cartridge for the air improving apparatus
Dispositif pour améliorer l'air pour l'utilisation dans des véhicules automobiles et cartouche pour le dispositif d'amélioration d'air

(30) Priorität: 25.06.2004 DE 202004010015 U
(43) Veröffentlichungstag der Anmeldung: 28.12.2005
(73) Patentinhaber: TRW Automotive Electronics & Components GmbH, 78315 Radolfzell (DE)
(72) Erfinder: Lauhoff, Dirk, 67659 Kaiserslautern (DE)
(74) Vertreter: Prinz & Partner

(56) Entgegenhaltungen:
- EP-A- 1 431 089
- US-A1- 2004 072 532
- PATENT ABSTRACTS OF JAPAN Bd. 013, Nr. 013 (M-783), 12. Januar 1989 (1989-01-12) & JP 63 222919 A (MATSUSHITA ELECTRIC WORKS LTD), 16. September 1988 (1988-09-16)

## Beschreibung

Die Erfindung betrifft eine Luftverbesserungsvorrichtung zur Verwendung in Kraftfahrzeugen sowie eine Kartusche für die Luftverbesserungsvorrichtung.

Luftverbesserungsvorrichtungen enthalten üblicherweise ein Parfum oder Duftöl, das langsam an die Umgebung abgegeben wird und so unerwünschte Gerüche überdeckt. Häufig bestehen diese Vorrichtungen aus einem AerosolBehälter, der winzige Tröpfchen einer Duftstofflösung in die Luft austreibt. Des weiteren werden Schalen mit gelatineartigen Massen eingesetzt, aus denen beim Trocknen und Schrumpfen die Duftstoffe an die Umgebung abgegeben werden. Des weiteren sind Luftverbesserungsvorrichtungen bekannt, die aus einem festen Träger, beispielsweise aus Pappe oder Kunststoff bestehen, der mit einem verdampfbaren Duftstoff getränkt oder beschichtet ist.

Derartige Luftverbesserungsvorrichtungen sind zur Verwendung in Kraftfahrzeugen nur bedingt geeignet. Sie sind im allgemeinen nicht in den Fahrzeugaufbau integriert, sondern lose im Fahrzeuginnenraum angeordnet, und stellen damit bei einem Fahrzeugunfall eine Verletzungsgefahr für die Fahrzeuginsassen dar. Bei Aerosolbehältem mit flüssigen Duftstofflösungen besteht grundsätzlich Bruchgefahr, so daß die Duftstofflösung in den Fahrzeuginnenraum auslaufen kann. Die übrigen Luftverbesserungsvorrichtungen sind im allgemeinen nicht verschließbar, so daß eine Regelung der Duftstoffabgabe nicht möglich ist. Beim Wechseln der Duftstoffträger kann es im übrigen zu einem Hautkontakt mit den Duftstoffen kommen.

In der EP 1 431 089 A1 ist eine Luftverbesserungsvorrichtung mit einem Gehäuse gezeigt, das in einem Fahrzeuginnenraumteil angeordnet und mit einer Belüftungsöffnung verbunden ist. In dem Gehäuse sind zwei mit Duftstoff gefüllte Kartuschen angeordnet. Jede Kartusche weist Abströmöffnungen auf, durch die der Duftstoff in jeweils einen Aufnahmeraum im Gehäuse austreten kann. Es ist ein Steuermechanismus mit einem beweglichen Steuerkranz vorgesehen, der wahlweise einen Aufnahmeraum mit der Belüftungsöffnung verbinden kann und somit ein Austreten von Duftstoff in die Belüftungsöffnung ermöglicht.

Die Erfindung schafft demgegenüber eine Luftverbesserungsvorrichtung zur Verwendung in Kraftfahrzeugen, die in den Fahrzeugaufbau integriert ist, eine einfache Handhabung ermöglicht und bei der die Duftstoffabgabe regelbar ist. Darüber hinaus gestattet die erfindungsgemäße Luftverbesserungsvorrichtung eine Auswechslung des Duftstoffträgers ohne Hautkontakt.

Erfindungsgemäß wird hierzu eine Luftverbesserungsvorrichtung zur Verwendung in Kraftfahrzeugen bereitgestellt, mit einem Gehäuse und einer in das Gehäuse eingesetzten Kartusche zur Aufnahme eines Duftstoffträgers, wobei das Gehäuse rückseitig an einer Instrumententafel des Kraftfahrzeugs angeordnet ist und wenigstens eine Durchströmöffnung aufweist, die mit einer Belüftungsöffnung in der Instrumententafel in Strömungsverbindung steht, und wobei die Kartusche wenigstens eine mit der Durchströmöffnung im Gehäuse korrespondierende Abströmöffnung aufweist und so im Gehäuse angeordnet ist, daß die Kartusche von einer geschlossenen Stellung, in der die Abströmöffnung vollständig bedeckt ist, in eine offene Stellung beweglich ist, in der die Durchströmöffnung über der Abströmöffnung liegt.

Die erfindungsgemäße Luftverbesserungsvorrichtung ermöglicht, daß ein mit einem Parfum oder einem anderen Duftstoff getränkter fester Duftstoffträger innerhalb einer Kartusche geliefert und ins Fahrzeug eingebaut werden kann. Nach einem Verbrauch des Parfums oder Duftstoffs kann die gesamte Kartusche ausgewechselt werden, so daß kein Hautkontakt mit dem Parfum oder Duftstoff hergestellt werden muß. Da die Kartusche beweglich ist, kann die Duftstoffabgabe durch den Fahrzeuginsassen beeinflußt werden. Außerdem ist eine Regelung der Duftstoffabgabe durch Einstellung von Positionen der Duftstoffkartusche zwischen der geschlossenen Stellung und der offenen Stellung möglich. In der geschlossenen Stellung ist dagegen die Abströmöffnung in der Kartusche verschlossen, so daß die Duftstoffabgabe durch den Fahrzeuginsassen auch vollständig unterbunden werden kann.

Bei einer bevorzugten Ausführungsform ist die Kartusche im Gehäuse drehbeweglich angeordnet. Dies ermöglicht eine einfache und optisch ansprechende Gestaltung der Luftverbesserungsvorrichtung. Hierzu umfaßt die Kartusche vorzugsweise ein zylinderförmiges Kartuschengehäuse mit radial gegenüberliegenden sich in Längsrichtung der Kartusche erstreckenden Abströmöffnungen. Ganz besonders bevorzugt sind die Abströmöffnungen im wesentlichen über die Gesamtlänge der Kartusche angeordnet und durch Stege voneinander getrennt. Hierdurch stehen besonders große Abströmöffnungen zur Verfügung, die eine variable Einstellung der Duftstoffabgabe in den Fahrzeuginnenraum ermöglichen.

Das von der Instrumententafel abgewandte freie Ende des Kartuschengehäuses ist vorzugsweise mit einem Deckel verschlossen. Damit ist der in der Kartusche angeordnete Duftstoffträger sicher vor Beschädigungen und unbeabsichtigten Hautkontakten geschützt. Der Deckel kann mit dem Kartuschengehäuse beispielsweise über eine Rastverbindung fest verbunden sein. Bei einer besonders bevorzugten Ausführungsform ist vorgesehen, daß der Deckel entweder manuell oder mit einem Werkzeug zerstörungsfrei abnehmbar ist. Dies ermöglicht einen einfachen Austausch des verbrauchten Duftstoffträgers und damit die Bereitstellung einer recyclingfähigen Duftstoffkartusche. An seinem der Instrumententafel zugewandten freien Ende weist das Kartuschengehäuse vorzugsweise einen Endabschnitt auf, der fest mit einem den Endabschnitt übergreifenden Kopfteil verbunden ist. Die Verbindung zwischen Kartuschengehäuse und Kopfteil kann beispielsweise dadurch erfolgen, daß im Endabschnitt wenigstens eine Ausnehmung, beispielsweise ein Längsschlitz, vorgesehen ist, in die ein Vorsprung im Kopfteil eingreift, so daß das den Endabschnitt übergreifende Kopfteil fest auf den Endabschnitt aufgeklemmt ist.

Das Kopfteil hat bevorzugt einen zylindrischen Abschnitt und einen radial vom zylindrischen Abschnitt vorstehen Flanschabschnitt und ist mit dem zylindrischen Abschnitt und dem daran befestigten Kartuschengehäuse durch eine Öffnung in der Instrumententafel geführt, so daß der Flanschabschnitt vorderseitig an die Instrumententafel angrenzt und das Kartuschengehäuse in das hinter der Öffnung liegende Gehäuse eingesetzt ist. Der zylindrische Abschnitt kann an seinem dem Kartuschengehäuse zugewandten Ende wenigstens einen radial vorstehenden Befestigungspunkt aufweisen, der von einem auf das Kartuschengehäuse wirkenden Federelement gegen die vom Fahrzeuginnenraum abgewandte Rückseite der Instrumententafel gedrückt wird. Zusätzlich kann auf einer dem Befestigungspunkt gegenüberliegenden Stelle des Flanschabschnitts eine Markierung angeordnet sein. Auf diese Weise wird ein einfacher Zugriff des Fahrzeuginsassen auf die Luftverbesserungsvorrichtung ermöglicht. An der Markierung auf dem Flanschabschnitt ist die jeweilige Position der Duftstoffkartusche jederzeit zu erkennen. Über den Befestigungspunkt ist das Kopfteil und die Duftstoffkartusche sicher an der Instrumententafel fixiert. Aus optischen Gründen kann der in den Fahrzeuginnenraum hineinragende zylindrische Abschnitts des Kopfteils noch mit einer Verschlußkappe versehen sein, die an die Struktur der Instrumententafel angepaßt werden kann. Wenn die Verschlußkappe den Flanschabschnitt überdeckt, kann die Verschlußkappe eine Markierung tragen, die mit der Markierung am Flanschabschnitt korrespondiert.

Das die Duftstoffkartusche aufnehmende Gehäuse weist eine stirnseitige Öffnung auf, die mit der Öffnung in der Instrumententafel korrespondiert und in die die Duftstoffkartusche, vom Fahrzeuginnenraum aus, eingesetzt ist. Dies ermöglicht ein einfaches Auswechseln der Duftstoffkartusche, sobald der Duftstoff verbraucht ist.

Zur besseren Abdichtung kann das Kartuschengehäuse außenseitig angeordnete Dichtlippen umfassen, die auf die Kartusche aufgespritzt sein können und die die Abströmöffnung in der geschlossenen Stellung gegen die Durchströmöffnung im Gehäuse zuverlässig abdichten.

Gegenstand der Erfindung ist weiter eine auswechselbare Kartusche mit den Merkmalen von Anspruch 21, zur Verwendung in einer Luftverbesserungsvorrichtung der oben beschriebenen Art. Vorteilhafte Ausgestaltungen der Luftverbesserungsvorrichtung und der Kartusche sind in den Unteransprüchen angegeben.

Die Erfindung wird nachfolgend anhand bevorzugter Ausführungsformen unter Bezugnahme auf die beigefügte Zeichnung beschrieben. In der Zeichnung zeigen:
- Figur 1 eine schematische Ansicht der erfindungsgemäßen Luftverbesserungsvorrichtung;
- Figur 2 eine perspektivische Ansicht von Einzelteilen der Luftverbesserungsvorrichtung aus Figur 1;
- Figur 3 eine schematische Ansicht der Luftverbesserungsvorrichtung aus Figur 1, gesehen von der Rückseite der Instrumententafel;
- Figur 4 eine schematische Rückansicht der Luftverbesserungsvorrichtung aus Figur 1, ohne Gehäuse;
- Figur 5a, 5b und 5c schematische Ansichten der Luftverbesserungsvorrichtung aus Figur 1 in einer geschlossenen Stellung, einer Zwischenstellung und einer offenen Stellung;
- Figur 6 eine perspektivische Ansicht von Einzelteilen der Luftverbesserungsvorrichtung gemäß einer weiteren Ausführungsform;
- Figur 7 eine perspektivische Ansicht der Luftverbesserungsvorrichtung aus Figur 6 in zusammengebauten Zustand;
- Figur 8 eine Schnittansicht der Luftverbesserungsvorrichtung aus Figur 6;
- Figur 9 eine weitere Schnittansicht der Luftverbesserungsvorrichtung aus Figur 6; und
- Figur 10a, 10b und 10c Schnittansichten der Luftverbesserungsvorrichtung aus Figur 6 in verschiedenen Stellungen.

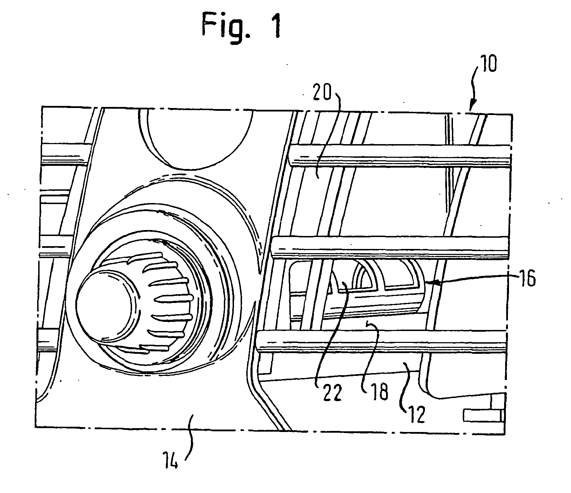

Die in Figur 1 in schematischer Ansicht gezeigte Luftverbesserungsvorrichtung 10 für Kraftfahrzeuge umfaßt ein Gehäuse 12, das an der dem Fahrzeuginnenraum abgewandten Rückseite einer Instrumententafel 14 oder einem Anbauteil angeordnet ist. In das Gehäuse 12 ist eine Kartusche 16 zur Aufnahme eines Duftstoffträgers (hier nicht dargestellt) eingesetzt. Der Duftstoffträger ist üblicherweise ein Feststoff oder Formkörper, der mit einem Duftöl oder Parfum getränkt ist. Das Gehäuse 12 weist wenigstens eine, hier seitlich angeordnete Durchströmöffnung 18 auf, die mit einer Belüftungsöffnung 20 in der Instrumententafel 12 und dem Innenraum des Kraftfahrzeugs in Strömungsverbindung steht. Die Kartusche 16 hat eine mit der Durchströmöffnung 18 im Gehäuse 12 korrespondierende Abströmöffnung 22 und ist so im Gehäuse 12 angeordnet, daß die Kartusche von einer geschlossenen Stellung, in der die Abströmöffnung 22 vollständig vom Gehäuse 12 bedeckt ist, in eine offene Stellung beweglich ist, in der die Durchströmöffnung 18 über der Abströmöffnung 22 liegt und somit eine Abgabe des Duftstoffs von dem in der Kartusche 16 enthaltenen Duftstoffträger in den Fahrzeuginnenraum ermöglicht.

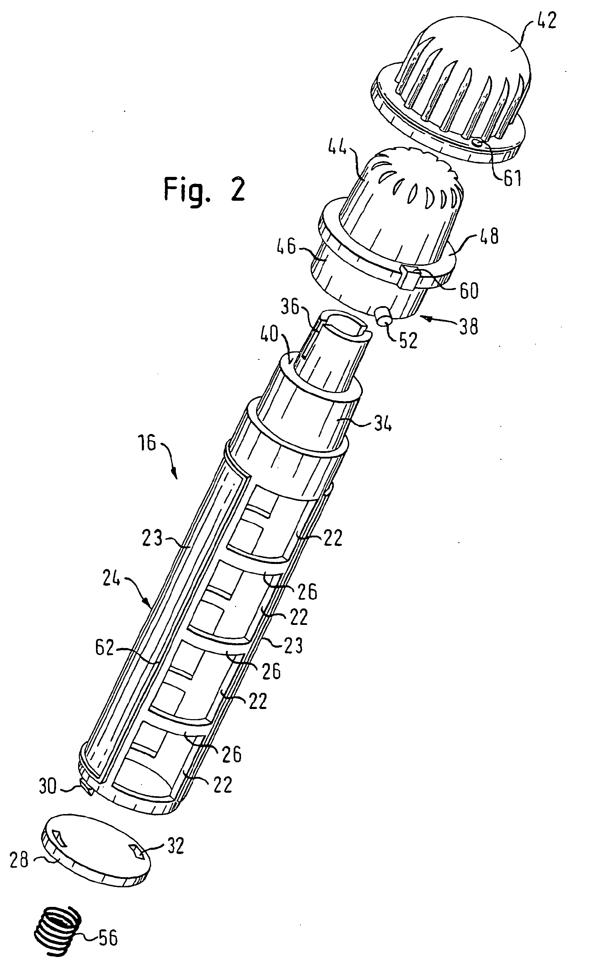

Die in Figur 2 in Einzelteilen in perspektivischer Ansicht gezeigte Kartusche 16 hat ein zylinderförmiges Kartuschengehäuse 24, in dem die Abströmöffnungen 22 axial nebeneinander angeordnet sind. Bei der hier gezeigten Ausführungsform sind die Abströmöffnungen im wesentlichen über die Gesamtlänge der Kartusche 16 bzw. des Kartuschengehäuses 24 verteilt und durch Stege 26 voneinander getrennt. Die Stege tragen zur Stabilität des Kartuschengehäuses bei. Das Kartuschengehäuse 16 ist darüber hinaus symmetrisch aufgebaut, d. h. es sind einander radial gegenüberliegende Paare von Abströmöffnungen 22 vorhanden. Zwischen den Paaren von Abströmöffnungen 22 befindet sich jeweils ein geschlossener Wandabschnitt 23 des Kartuschengehäuses 24. Bei der bevorzugten drehbeweglichen Anordnung der Kartusche 16 im Gehäuse 12 kann somit ein einfacher Übergang von der geschlossenen Stellung der Kartusche 16 in die offene Stellung erreicht werden.

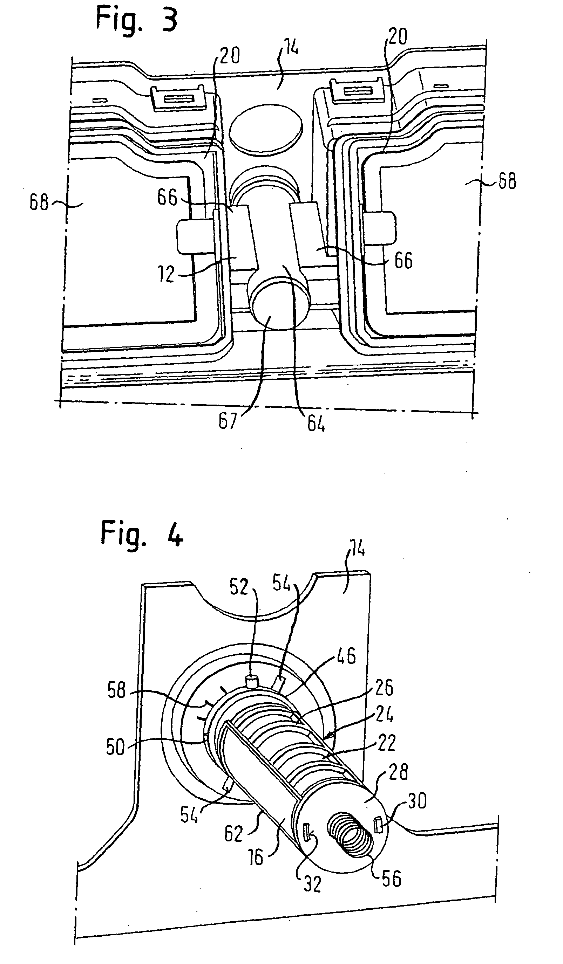

An seinem im eingebauten Zustand (Figur 4) von der Instrumententafel abgewandten Ende ist das Kartuschengehäuse 24 mit einem Deckel 28 verschlossen. Die Verbindung zwischen dem Deckel 28 und dem Kartuschengehäuse 24 erfolgt bei der hier gezeigten Ausführungsform über eine Rastverbindung durch an das Ende des Kartuschengehäuses angeformt Rasthaken 30, die in entsprechende Öffnungen 32 eingreifen und den Deckel am Kartuschengehäuse 24 festlegen. Die Rastverbindung ist dabei so ausgelegt, daß der Deckel 28 für einen Austausch des in das Kartuschengehäuse 24 eingebrachten Duftstoffträgers manuell oder mittels eines Werkzeugs geöffnet werden kann. Hierdurch wird die Wiederverwertbarkeit der Kartusche 16 nach dem Verbrauch des Duftstoffträgers erreicht, da der Deckel 28 zerstörungsfrei abgenommen werden kann.

An seinem dem Deckel 28 gegenüberliegenden Ende weist das Kartuschengehäuse 24 einen angeformten zylindrischen Endabschnitt 34 auf, in den ein Längsschlitz 36 eingebracht ist. Der Endabschnitt 34 hat einen im Vergleich zum Kartuschengehäuse 24 geringeren Durchmesser. Im eingebauten Zustand der Kartusche 16 übergreift ein Kopfteil 38 den Endabschnitt 34, wobei ein im Kopfteil 38 angeordneter Vorsprung oder Zapfen in die Längsschlitze 36 am Endabschnitt 34 eingreift, so daß eine feste Verbindung zwischen Kopfteil 38 und dem Endabschnitt 34 des Kartuschengehäuses 24 besteht. Bei der hier gezeigten Ausführungsform weist der Endabschnitt 34 weiter einen Schulterabschnitt 40 auf der an einen Anschlag (hier nicht dargestellt) im Kopfteil 38 angrenzt. Auf das Kopfteil 38 ist ferner eine Verschlußkappe 42 aufgesetzt, die an ihrer Innenfläche mit hier nicht dargestellten Vorsprüngen versehen sein kann, welche in korrespondierende Aussparungen 44 an der Stirn- oder Seitenfläche des Kopfteils 38 eingreifen können und so eine bessere Kraftübertragung von der Verschlußkappe 42 auf das Kopfteil 38 bzw. das mit dem Kopfteil verbundene Kartuschengehäuse 24 ermöglichen. Alternativ dazu können das Kopfteil 38 und die Verschlußkappe 42 auch einstückig miteinander, beispielsweise als Zweikomponenten-Spritzteil, gebildet sein.

Das Kopfteil 38 weist einen zylindrischen Abschnitt 46 und einen vom zylindrischen Abschnitt radial vorstehenden Flanschabschnitt 48 auf. Wie in Figur 4 zu erkennen ist, ist der zylindrische Abschnitt 46 mit der darin über den Endabschnitt 34 befestigten Kartusche 16 durch eine Öffnung 50 in der Instrumententafel 14 geführt und grenzt mit zwei radial vorstehenden Befestigungspunkten 52, die an dem dem Kartuschengehäuse 24 zugewandten Ende angeformt sind, an die Rückseite der Instrumententafel an. Das Einführen der Kartusche 16 mit den Befestigungspunkten 52 am Kopfteil 38 in die Öffnung 50 der Instrumententafel 14 wird durch Aussparungen 54 am Rand der Öffnung 50 gestattet, die in Größe und Form den Befestigungspunkten 52 entsprechen. An der vorderen Seite der Instrumententafel 14 werden die Aussparungen 54 durch den an die Instrumententafel 14 angrenzenden Flanschabschnitt 48 verdeckt.

Im eingebauten Zustand wird die Kartusche 16 durch ein in das Gehäuse 12 eingebrachtes Federelement 56, beispielsweise eine Spiralfeder oder eine Blattfeder, gegen die Instrumententafel 14 mit Kraft beaufschlagt. Dadurch werden die Befestigungspunkte 52 am zylindrischen Abschnitt 46 des Kopfteils 38 gegen die Rückseite der Instrumententafel 14 gedrückt, und die Kartusche 16 wird ähnlich wie mit einem Bajonettverschluß sicher in Position gehalten. An der Rückseite der Instrumententafel 14 können zudem noch Rastpunkte 58 vorgesehen sein in welche die Befestigungspunkte 52 bei der Betätigung des Kopfteils 38 durch die Verschlußkappe 42 einrasten und somit vorbestimmte Positionen der Kartusche 16 und der Abströmöffnungen 22 definieren.

An der einem Befestigungspunkt 52 gegenüberliegenden Stelle des Flanschabschnitts 48 im Kopfteil 38 ist darüber hinaus eine Markierung 60 in Form eines radial und/oder axial vorstehenden Vorsprungs vorgesehen, der in eine entsprechende, in die Verschlußkappe 42 eingebrachte Aussparung (hier nicht gezeigt) eingreift, deren Position mit einer Markierung 61 versehen ist. Über die Markierungen 60 bzw. 61 kann somit der Fahrzeuginsasse die Stellung der Kartusche 16 und der Abströmöffnungen 22 sicher erkennen. Beispielweise kann die nach oben zeigende Markierung 61 die geschlossene Stellung der Kartusche 16 anzeigen, in der die Abströmöffnungen 22 vollständig vom Gehäuse 12 bedeckt sind. Die offene Stellung der Kartusche 16 mit freiliegenden Abströmöffnungen 22 wird dementsprechend durch eine Drehung der Verschlußkappe um 90 Grad im Uhrzeigersinn und eine dann entsprechend nach rechts weisende Markierung 61 angezeigt.

Zur besseren Abdichtung der Abströmöffnungen 22 gegenüber dem Fahrzeuginnenraum ist das Kartuschengehäuse 24 mit einer Dichtlippe 62 versehen, die in Längsrichtung an die Abströmöffnung 22 angrenzt und diese in der geschlossenen Stellung zuverlässig vom Fahrzeuginnenraum abdichtet, so daß keine unbeabsichtigte Abgabe von Duftstoffen aus dem in der Kartusche 16 enthaltenen Duftstoffträger erfolgt.

Das in Figur 3 in rückseitiger Ansicht gezeigte Gehäuse 12 weist einen hohlzylindrischen Abschnitt 64 auf, in den die Kartusche 16 drehbeweglich aufgenommen ist, sowie seitlich vom hohlzylindrischen Abschnitt 64 abstehende rohrförmige Ansatzstücke 66, die eine Strömungsverbindung zwischen den Belüftungsöffnungen 20 in der Instrumententafel 14 und den Abströmöffnungen 22 in der Kartusche 16 herstellen. Das Gehäuse hat ferner einen geschlossenen Bodenabschnitt 67, an dem sich das Federelement 56 der Kartusche 16 abstützt. Die Belüftungsöffnungen 20 sind rückseitig in bekannter Weise mit Lüftungsklappen 68 versehen, die eine regelbare Luftzufuhr zum Fahrzeuginnenraum gestatten. Das Gehäuse 12 kann in die Instrumententafel 14 oder ein entsprechendes Anbauteil integriert oder vorzugsweise als separates Bauteil ausgeführt sein.

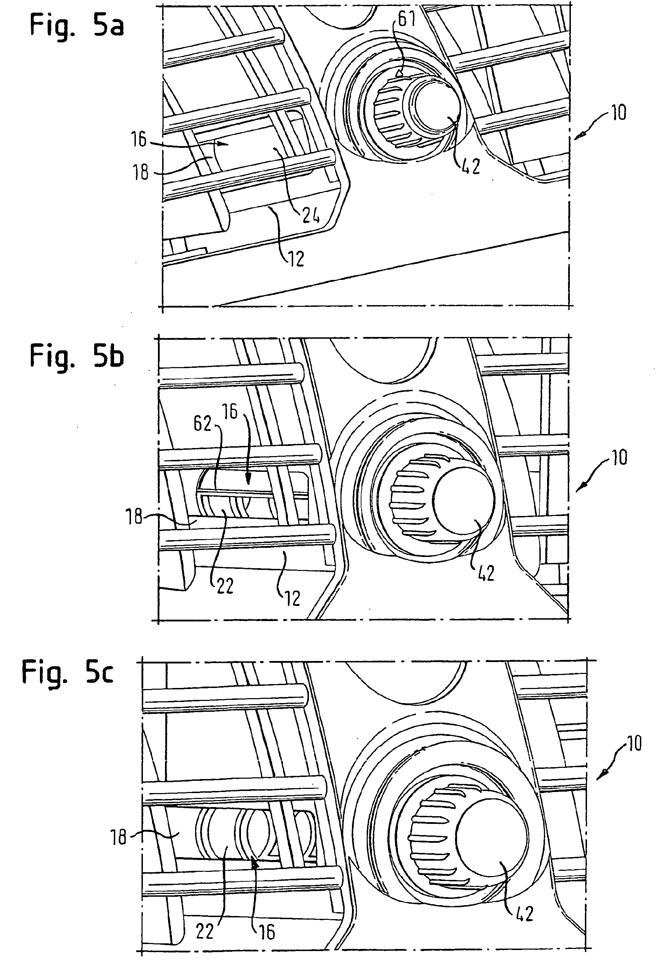

Im Folgenden soll die Funktionsweise der erfindungsgemäßen Luftverbesserungsvorrichtung 10 anhand der Figuren 5a bis 5c näher erläutert werden.

Figur 5a zeigt die Luftverbesserungsvorrichtung 10 in der geschlossenen Stellung. Die in das Gehäuse 12 eingesetzte Kartusche 16 mit dem darin enthaltenen Duftstoffträger ist so angeordnet, daß die Abströmöffnungen 22 vollständig vom Gehäuse 12 bedeckt sind. Die Durchströmöffnungen 18 im Gehäuse 12 sind hier von dem geschlossenen Wandbereich 23 des Kartuschengehäuses 24 verdeckt. Die sich in axialer Richtung erstreckenden Dichtlippen 62 dichten die Abströmöffnungen 22 bzw. die Durchströmöffnungen 18 vollständig vom Fahrzeuginnenraum ab, so daß keine Abgabe von Duftstoffen in den Fahrzeuginnenraum erfolgen kann. Die an der Kappe 42 angebrachte Markierung 62 zeigt in Figur 5a nach oben und läßt somit die geschlossene Stellung der Kartusche 16 erkennen.

Durch eine Drehung der Kappe 42 um ca. 20° nach rechts wird die in Figur 5b erreichte Zwischenstellung erreicht, bei der die Abströmöffnungen 22 noch teilweise vom Gehäuse 12 verdeckt werden. Da in dieser Stellung ein geringerer Strömungsquerschnitt zur Verfügung steht, ist die Abgabe von Duftstoffen in den Fahrzeuginnenraum reduziert.

Bei der in Figur 5c gezeigten offenen Stellung der Kartusche 16 liegt die Durchströmöffnung 18 vollständig über den Abströmöffnungen 22. In dieser Stellung ist der größtmögliche Strömungsquerschnitt erreicht und die Abgabe des Duftstoffs in den Fahrzeuginnenraum erfolgt mit größter Intensität.

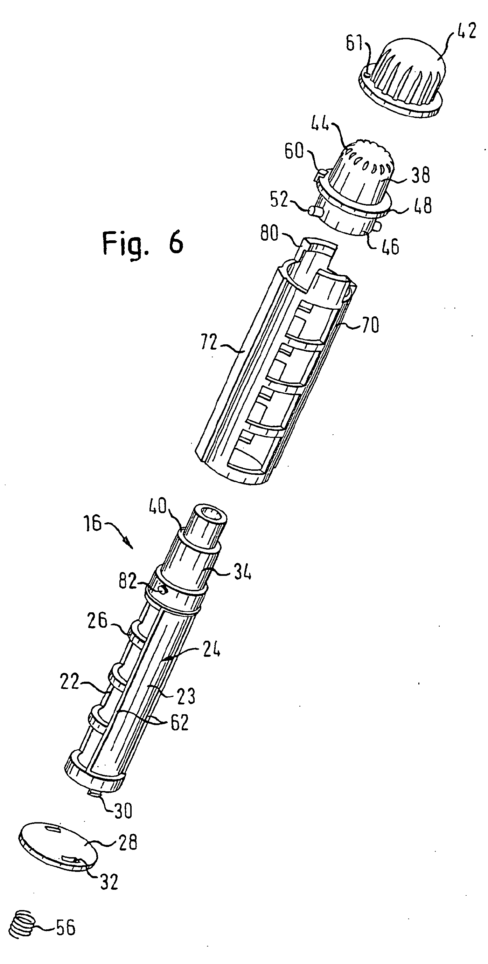

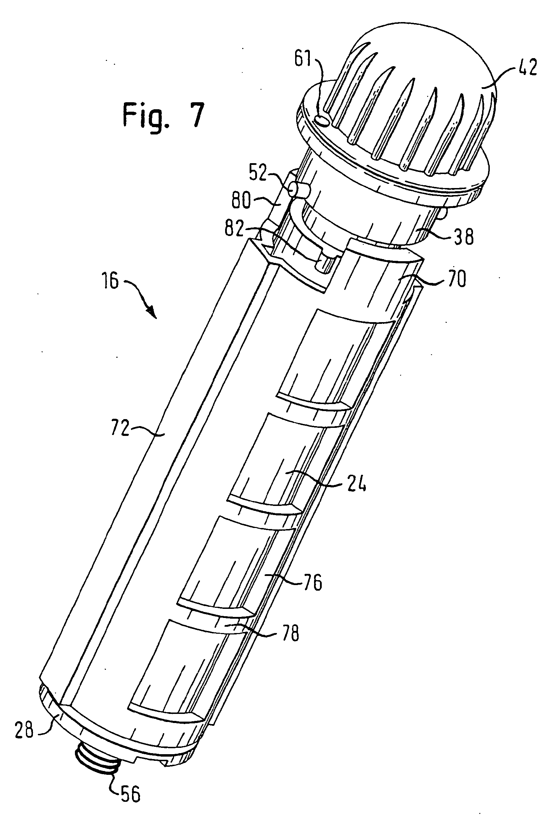

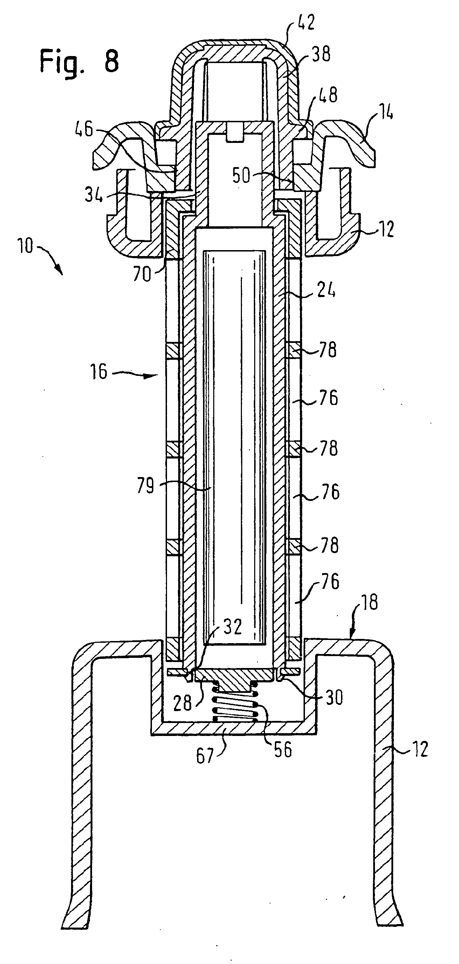

Eine weitere Ausführungsform der erfindungsgemäßen Luftverbesserungsvorrichtung 10 ist in den Figuren 6 bis 9 dargestellt. Bauteile, welche die gleiche Funktion wie in der oben beschriebenen ersten Ausführungsform der erfindungsgemäßen Luftverbesserungsvorrichtung 10 erfüllen, sind mit den gleichen Bezugszeichen versehen. Insoweit wird auf die obige Beschreibung verwiesen.

Bei der hier dargestellten Ausführungsform der erfindungsgemäßen Luftverbesserungsvorrichtung 10 umfaßt die Kartusche 16 das zylinderförmige Kartuschengehäuse 24 sowie zusätzlich eine Aufnahmehülse 70 für das Kartuschengehäuse 24. Die Aufnahmehülse 70 ist an ihrem Außenumfang mit einer Längsrippe 72 versehen, die im eingebauten Zustand in eine korrespondierende Nut 74 im Gehäuse 12 eingreift und die Aufnahmehülse 70 unbeweglich im Gehäuse 12 fixiert. Bevorzugt sind an der Aufnahmehülse 70 zwei einander gegenüberliegende Längsrippen 72 und im Gehäuse 12 zwei entsprechende Nuten 74 ausgebildet. Die Aufnahmehülse 70 umfaßt ferner wenigstens eine sich in Längsrichtung erstreckende Durchtrittsöffnung 76, die durch Stege 78 in mehrere Öffnungsabschnitte unterteilt sein kann. Vorzugsweise sind in der Aufnahmehülse 70 zwei einander gegenüberliegende Durchtrittsöffnungen 76 ausgebildet, die mit den Abströmöffnungen 22 im Kartuschengehäuse 24 korrespondieren und im eingebauten Zustand außerdem fluchtend zur Durchströmöffnung 18 im Gehäuse 12 angeordnet sind.

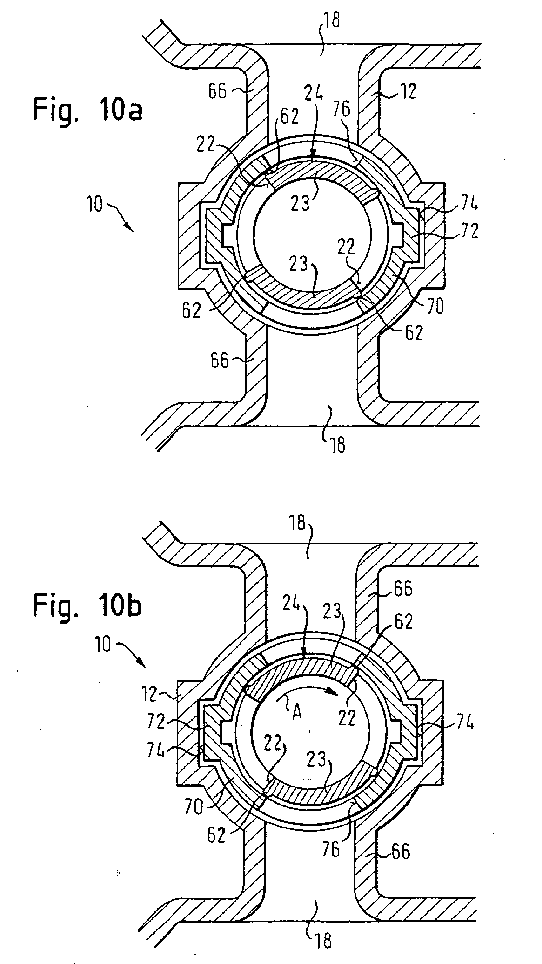

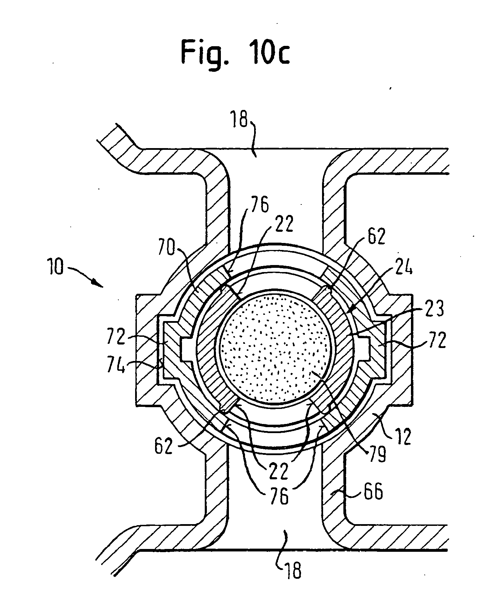

Das zylinderförmige Kartuschengehäuse 24 ist drehbeweglich in der Aufnahmehülse 70 angeordnet und kann von der geschlossenen Stellung, in der die Abströmöffnungen 22 des Kartuschengehäuses 24 vollständig, hier von einem durchgehenden Wandabschnitt der Aufnahmehülse 70, bedeckt sind, in eine offene Stellung bewegt werden, in der die Durchtrittsöffnung 76 über den Abströmöffnungen 22 liegt und damit die Abgabe eines Duftstoffs von dem im Kartuschengehäuse 24 aufgenommenen Duftstoffträger 79 (Figur 10) in den Fahrzeuginnenraum ermöglicht. Zur Begrenzung der Drehbewegung des Kartuschengehäuses 24 in der Aufnahmehülse 70 und zur Definition der offenen Stellung und der geschlossenen Stellung kann die Aufnahmehülse 70 schließlich an einem ihrer freien Enden einen Anschlag 80 aufweisen, der mit einer nahe des Endabschnitts 34 am Kartuschengehäuse 24 angeordneten, radial vorstehenden Nase 82 zusammenwirkt.

In den Figuren 10a bis 10c ist die Funktionsweise der Luftverbesserungsvorrichtung 10 gemäß der hier beschriebenen Ausführungsform dargestellt. Die Parfumkartusche 16 wird in ihrer geschlossenen Stellung durch eine Öffnung in der Instrumententafel (hier nicht dargestellt) in das Gehäuse 12 eingesetzt. In der geschlossenen Stellung sind die Abströmöffnungen 22 von den durchgehenden Wandabschnitten der Aufnahmehülse 70 vollständig bedeckt. Des weiteren liegt die Wand 23 des Kartuschengehäuses 24 vor den Durchtrittsöffnungen 76 in der Aufnahmehülse 70. Außerdem ist das Kartuschengehäuse 24 gegenüber der Aufnahmehülse durch die Dichtlippen 62 so abgedichtet, daß kein Duftstoff aus dem im Kartuschengehäuse 64 angeordneten Duftstoffträger über die Durchströmöffnungen 18 im Gehäuse 12 in das Fahrzeuginnere gelangen kann.

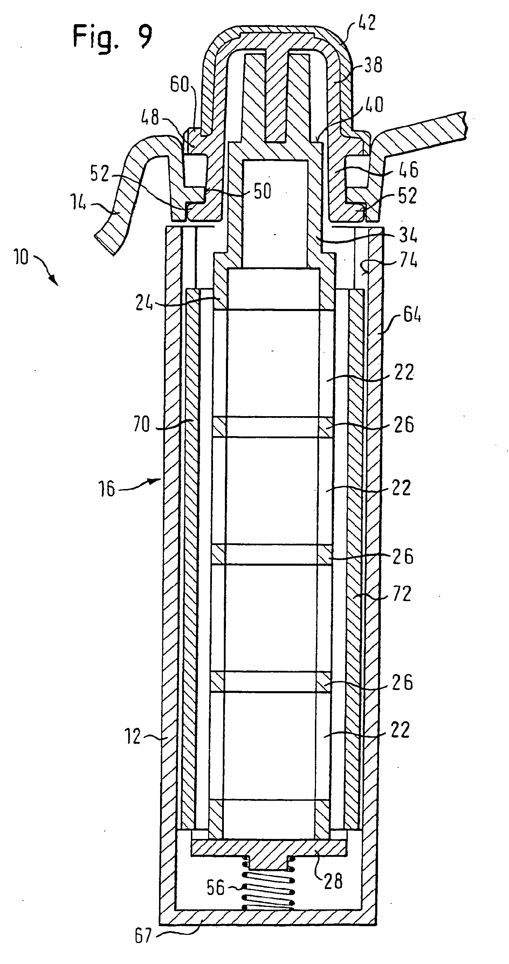

Durch eine geringfügige Drehung in Richtung des Pfeils A (Figur 10b) wird der Befestigungspunkt 52 (Figur 9) rückseitig in Anlage an die Instrumententafel 14 gebracht und die Kartusche 16 wie bei einem Bajonettverschluß verrastet. Auch in dieser Position befindet sich die Kartusche 16 noch in der geschlossenen Stellung. Durch Drücken und Drehen des für den Fahrzeuginsassen zugänglichen, aus Kopfteil 38 und Verschlußkappe 42 gebildeten Bedienknopfes kann die Kartusche 16 so nach einem Verbrauch des Duftstoffes leicht wieder entfernt und durch eine neue Kartusche 16 ersetzt werden.

Durch eine weitere Drehung in Richtung des Pfeils A (Figur 10b) werden schließlich die Abströmöffnungen 22 freigegeben (Figur 10c) und der Duftstoff kann von dem Duftstoffträger 79 aus den Abströmöffnungen 22 und der darüber liegenden Durchtrittsöffnung 76 über die Durchströmöffnung 18 im Gehäuse 12 in das Fahrzeuginnere abgegeben werden. Die Endposition dieser offenen Stellung der Kartusche 16 kann durch den Anschlag 80 an der Aufnahmehülse 70 und die Nase 82 am Kartuschengehäuse 24 (Figur 9) festgelegt werden. Über Zwischenstellungen zwischen der geschlossenen und der offenen Stellung läßt sich die in den Fahrzeuginnenraum abgegebene Menge des Duftstoffs nach den Wünschen des Fahrzeuginsassen regulieren.

## Patentansprüche

1. Luftverbesserungsvorrichtung (10) zur Verwendung in Kraftfahrzeugen, mit einem Gehäuse (12) und einer in das Gehäuse (16) eingesetzten Kartusche (16) zur Aufnahme eines Duftstoffträgers (79), wobei das Gehäuse (12) rückseitig an einer Instrumententafel (14) des Kraftfahrzeugs angeordnet ist und wenigstens eine Durchströmöffnung (18) aufweist, die mit Belüftungsöffnungen (20) in der Instrumententafel (14) in Strömungsverbindung steht, und wobei die Kartusche (16) wenigstens eine mit der Durchströmöffnung (18) im Gehäuse (12) korrespondierende Abströmöffnung (22) aufweist, wobei die Luftverbesserungsvorrichtung **dadurch gekennzeichnet ist, dass** die Kartusche derart im Gehäuse (12) angeordnet ist, daß die Kartusche (16) von einer geschlossenen Stellung, in der die Abströmöffnung (22) vollständig bedeckt ist, in eine offene Stellung beweglich ist, in der die Durchströmöffnung (18) über der Abströmöffnung (22) liegt.

2. Luftverbesserungsvorrichtung (10) nach Anspruch 1, **dadurch gekennzeichnet, daß** die Kartusche (16) im Gehäuse (12) drehbeweglich angeordnet ist.

3. Luftverbesserungsvorrichtung (10) nach Anspruch 1 oder 2, **dadurch gekennzeichnet, daß** die Kartusche (16) ein zylinderförmiges Kartuschengehäuse (24) umfaßt.

4. Luftverbesserungsvorrichtung (10) nach einem der Ansprüche 1 bis 3, **dadurch gekennzeichnet, daß** die Kartusche (16) radial gegenüberliegende, sich in Längsrichtung der Kartusche (16) erstreckende Abströmöffnungen (22) aufweist.

5. Luftverbesserungsvorrichtung (10) nach einem der Ansprüche 1 bis 4, **dadurch gekennzeichnet, daß** die Abströmöffnungen (22) im wesentlichen über die Gesamtlänge der Kartusche (16) angeordnet und durch Stege (26) voneinander getrennt sind.

6. Luftverbesserungsvorrichtung (10) nach einem der Ansprüche 1 bis 5, **dadurch gekennzeichnet, daß** das von der Instrumententafel (14) abgewandte Ende der Kartusche (16) einen Deckel (28) umfaßt.

7. Luftverbesserungsvorrichtung (10) nach Anspruch 6, **dadurch gekennzeichnet, daß** der Deckel (28) zerstörungsfrei abnehmbar ist.

8. Luftverbesserungsvorrichtung (10) nach einem der Ansprüche 1 bis 7, **dadurch gekennzeichnet, daß** die Kartusche (16) einen Endabschnitt (34) aufweist, der fest mit einem Kopfteil (38) verbunden ist.

9. Luftverbesserungsvorrichtung (10) nach Anspruch 8, **dadurch gekennzeichnet, daß** der Endabschnitt (34) der Kartusche (16) eine Ausnehmung (36) aufweist, in die ein Vorsprung im Kopfteil (38) eingreift, so daß das Kopfteil (38) auf den Endabschnitt (34) aufgeklemmt ist.

10. Luftverbesserungsvorrichtung (10) nach Anspruch 8 oder 9, **dadurch gekennzeichnet, daß** das Kopfteil (38) einen zylindrischen Abschnitt (46) und einen radial vom zylindrischen Abschnitt (46) vorstehenden Flanschabschnitt (48) aufweist und mit dem zylindrischen Abschnitt (46) durch eine Öffnung (50) in der Instrumententafel (14) geführt ist, so daß der Flanschabschnitt (48) vorderseitig an die Instrumententafel (14) angrenzt.

11. Luftverbesserungsvorrichtung (10) nach Anspruch 10, **dadurch gekennzeichnet, daß** der zylindrische Abschnitt (46) an seinem der Kartusche (16) zugewandten Ende wenigstens einen radial vorstehenden Befestigungspunkt (52) aufweist, der von einem auf die Kartusche (16) wirkenden Federelement (56) gegen die Rückseite der Instrumententafel (16) gedrückt wird.

12. Luftverbesserungsvorrichtung (10) nach Anspruch 11, **dadurch gekennzeichnet, daß** auf einer dem Befestigungspunkt (52) gegenüberliegenden Stelle des Flanschabschnitts (48) eine Markierung (60) angeordnet ist.

13. Luftverbesserungsvorrichtung (10) nach einem der Ansprüche 10 bis 12, **dadurch gekennzeichnet, daß** das der zylindrische Abschnitt (46) an seinem der Kartusche (16) abgewandten Ende mit einer Kappe (42) versehen ist.

14. Luftverbesserungsvorrichtung (10) nach einem der Ansprüche 1 bis 13, **dadurch gekennzeichnet, daß** das Gehäuse (12) eine stirnseitige Öffnung umfaßt, die mit einer Öffnung (50) in der Instrumententafel (14) korrespondiert und in die die Kartusche (16) auswechselbar eingesetzt ist.

15. Luftverbesserungsvorrichtung (10) nach einem der Ansprüche 1 bis 14, **dadurch gekennzeichnet, daß** die Kartusche (16) außenseitig angeordnete Dichtlippen (62) umfaßt, die die Abströmöffnungen (22) in der geschlossenen Stellung gegen die Durchströmöffnung (18) im Gehäuse (12) abdichten.

16. Luftverbesserungsvorrichtung (10) nach einem der Ansprüche 1 bis 15, **dadurch gekennzeichnet, daß** die Kartusche (16) ein zylinderförmiges Kartuschengehäuse (24) und eine Aufnahmehülse (70) für das Kartuschengehäuse (24) umfaßt, wobei die Aufnahmehülse (70) gegenüber dem Gehäuse (12) unbeweglich angeordnet und das Kartuschengehäuse (24) in der Aufnahmehülse (70) drehbeweglich angeordnet ist.

17. Luftverbesserungsvorrichtung (10) nach Anspruch 16, **dadurch gekennzeichnet, daß** die Aufnahmehülse (70) an ihrem Außenumfang wenigstens eine Längsrippe (72) aufweist, die in eine korrespondierende Nut (74) im Gehäuse (12) eingreift und die Aufnahmehülse (70) im Gehäuse (12) fixiert.

18. Luftverbesserungsvorrichtung (10) nach Anspruch 16 oder 17, **dadurch gekennzeichnet, daß** die Aufnahmehülse (70) wenigstens eine in Längsrichtung angeordnete Durchtrittsöffnung (76) aufweist, die fluchtend mit der Durchströmöffnung (18) im Gehäuse (12) angeordnet ist.

19. Luftverbesserungsvorrichtung (10) nach Anspruch 18, **dadurch gekennzeichnet, daß** die Durchtrittsöffnung (76) durch Stege (78) unterteilt ist.

20. Luftverbesserungsvorrichtung (10) nach einem der Ansprüche 16 bis 19, **dadurch gekennzeichnet, daß** die Aufnahmehülse (70) an ihrem der Instrumententafel (14) zugewandten Ende einen Anschlag (80) zur Begrenzung der Drehbewegung des Kartuschengehäuses (24) aufweist.

21. Kartusche (16) zur Verwendung in einer Luftverbesserungsvorrichtung (10) gemäß einem der vorhergehenden Ansprüche, mit einem zylinderförmigen Kartuschengehäuse (24) zur Aufnahme eines Duftstoffträgers und in Längsrichtung des Kartuschengehäuses (24) angeordneten Abströmöffnungen (22), **dadurch gekennzeichnet, daß** die Abströmöffnungen (22) im wesentlichen über die Gesamtlänge der Kartusche (16) angeordnet und durch Stege (26) voneinander getrennt sind.

22. Kartusche (16) nach Anspruch 21, **dadurch gekennzeichnet, daß** ein freies Ende des Kartuschengehäuses (24) mit einem Deckel (28) verschlossen ist.

23. Kartusche (16) nach Anspruch 22, **dadurch gekennzeichnet, daß** der Deckel (28) zerstörungsfrei abnehmbar ist.

24. Kartusche (16) nach Anspruch 22 oder 23, **dadurch gekennzeichnet, daß** das Kartuschengehäuse (24) an seinem dem Deckel (28) gegenüberliegenden Ende einen angeformten Endabschnitt (34) aufweist, der fest mit einem den Endabschnitt (34) übergreifenden Kopfteil (38) verbunden ist.

25. Kartusche (16) nach Anspruch 24, **dadurch gekennzeichnet, daß** der Endabschnitt (34) des Kartuschengehäuses (24) eine Ausnehmung (36) aufweist, in die ein Vorsprung im Kopfteil (38) eingreift, so daß das Kopfteil (38) auf den Endabschnitt (34) aufgeklemmt ist.

26. Kartusche (16) nach Anspruch 24 oder 25, **dadurch gekennzeichnet, daß** das Kopfteil (38) einen zylindrischen Abschnitt (46) und einen radial vom zylindrischen Abschnitt (46) vorstehenden Flanschabschnitt (48) aufweist.

27. Kartusche (16) nach Anspruch 26, **dadurch gekennzeichnet, daß** der zylindrische Abschnitt (46) an seinem dem Kartuschengehäuse (24) zugewandten Ende wenigstens einen radial vorstehenden Befestigungspunkt (52) zur Fixierung der Kartusche (16) aufweist.

28. Kartusche (16) nach Anspruch 27, **dadurch gekennzeichnet, daß** auf einer dem Befestigungspunkt (52) gegenüberliegenden Stelle des Flanschabschnitts (48) eine Markierung (60) angeordnet ist.

29. Kartusche (16) nach einem der Ansprüche 26 bis 28, **dadurch gekennzeichnet, daß** der zylindrische Abschnitt (46) an seinem dem Kartuschengehäuse (24) abgewandten Ende mit einer Kappe (42) versehen ist.

30. Kartusche (16) nach einem der Ansprüche 21 bis 29, **dadurch gekennzeichnet, daß** das Kartuschengehäuse (24) außenseitig angeordnete und in axialer Richtung verlaufende Dichtlippen (62) umfaßt.

31. Kartusche (16) nach einem der Ansprüche 21 bis 30, **dadurch gekennzeichnet, daß** die Kartusche (16) ferner eine Aufnahmehülse (70) für das Kartuschengehäuse (24) mit wenigstens einer in Längsrichtung angeordneten Durchtrittsöffnung (76) umfaßt, wobei das Kartuschengehäuse (24) in der Aufnahmehülse (70) drehbeweglich angeordnet und von einer geschlossenen Stellung, in der die Abströmöffnungen (22) vollständig von der Aufnahmehülse (70) bedeckt sind, in eine offene Stellung beweglich ist, in der die Durchtrittsöffnung (76) über den Abströmöffnungen (22) liegt.

32. Kartusche (16) nach Anspruch 31, **dadurch gekennzeichnet, daß** die Aufnahmehülse (70) an ihrem Außenumfang wenigstens eine Längsrippe (72) zur Fixierung der Aufnahmehülse (70) aufweist.

33. Kartusche (16) nach Anspruch 31 oder 32, **dadurch gekennzeichnet, daß** die Durchtrittsöffnung (76) durch Stege (78) unterteilt ist.

34. Kartusche (16) nach einem der Ansprüche 31 bis 33, **dadurch gekennzeichnet, daß** die Aufnahmehülse (70) an einem ihrer freien Enden einen Anschlag (80) zur Begrenzung der Drehbewegung des Kartuschengehäuses (24) aufweist.

## Claims

1. An air deodorizing device (10) for use in a motor vehicle, comprising a housing (12) and a cartridge (16) inserted in the housing (12) and adapted to accommodate a fragrance carrier (79), the housing (12) being arranged at the rear side of an instrument panel (14) of the motor vehicle and having at least one flow-through opening (18) which is in fluid communication with ventilation openings (20) in the instrument panel (14), and the cartridge (16) having at least one outflow opening (22) corresponding to the flow-through opening (18) in the housing (12), the air deodorizing device being **characterized in that** the cartridge is arranged in the housing (12) such that the cartridge (16) can be moved from a closed position, in which the outflow opening (22) is completely covered, to an open position, in which the flow-through opening (18) is situated over the outflow opening (22).

2. The air deodorizing device (10) as recited in claim 1, **characterized in that** the cartridge (16) is rotatably arranged in the housing (12).

3. The air deodorizing device (10) as recited in claim 1 or 2, **characterized in that** the cartridge (16) includes a cylindrical cartridge housing (24).

4. The air deodorizing device (10) as recited in any of claims 1 to 3, **characterized in that** the cartridge (16) includes outflow openings (22) that are radially opposite each other and extend in the longitudinal direction of the cartridge (16).

5. The air deodorizing device (10) as recited in any of claims 1 to 4, **characterized in that** the outflow openings (22) are arranged essentially over the entire length of the cartridge (16) and are separated from each other by crosspieces (26).

6. The air deodorizing device (10) as recited in any of claims 1 to 5, **characterized in that** the end of the cartridge (16) facing away from the instrument panel (14) has a cover (28).

7. The air deodorizing device (10) as recited in claim 6, **characterized in that** the cover (28) can be removed without being destroyed.

8. The air deodorizing device (10) as recited in any of claims 1 to 7, **characterized in that** the cartridge (16) has an end section (34) which is firmly connected to a head part (38).

9. The air deodorizing device (10) as recited in claim 8, **characterized in that** the end section (34) of the cartridge (16) has a cutout (36) in which a projection in the head part (38) engages, so that the head part (38) is clamped onto the end section (34).

10. The air deodorizing device (10) as recited in claim 8 or 9, **characterized in that** the head part (38) has a cylindrical section (46) and a flange section (48) that protrudes radially from the cylindrical section (46), and the head part is guided through an opening (50) in the instrument panel (14) by the cylindrical section (46), so that the flange section (48) adjoins the front side of the instrument panel (14).

11. The air deodorizing device (10) as recited in claim 10, **characterized in that** the cylindrical section (46) at its end facing the cartridge (16) has at least one radially protruding attachment point (52) which is pressed against the rear side of the instrument panel (14) by a spring element (56) that acts upon the cartridge (16).

12. The air deodorizing device (10) as recited in claim 11, **characterized in that** a marking (60) is arranged on a location of the flange section (48) that is situated opposite the attachment point (52).

13. The air deodorizing device (10) as recited in any of claims 10 to 12, **characterized in that** the cylindrical section (46) at its end facing away from the cartridge (16) is provided with a cap (42).

14. The air deodorizing device (10) as recited in any of claims 1 to 13, **characterized in that** the housing (12) includes an opening on the end face, which corresponds to an opening (50) in the instrument panel (14) and in which the cartridge (16) is replaceably inserted.

15. The air deodorizing device (10) as recited in any of claims 1 to 14, **characterized in that** the cartridge (16) has sealing lips (62) which are arranged on the exterior and which, in the closed position, seal off the outflow openings (22) from the flow-through opening (18) in the housing (12).

16. The air deodorizing device (10) as recited in any of claims 1 to 15, **characterized in that** the cartridge (16) has a cylindrical cartridge housing (24) and a receiving sleeve (70) for the cartridge housing (24), the receiving sleeve (70) being arranged so as to be immovable in relation to the housing (12), and the cartridge housing (24) being rotatably arranged in the receiving sleeve (70).

17. The air deodorizing device (10) as recited in claim 16, **characterized in that** the receiving sleeve (70) on its exterior circumference has at least one longitudinal rib (72) which engages in a corresponding groove (74) in the housing (12) and fixes the receiving sleeve (70) in position in the housing (12).

18. The air deodorizing device (10) as recited in claim 16 or 17, **characterized in that** the receiving sleeve (70) has at least one through opening (76) which is arranged in the longitudinal direction and which is arranged so as to be aligned with the flow-through opening (18) in the housing (12).

19. The air deodorizing device (10) as recited in claim 18, **characterized in that** the through opening (76) is subdivided by crosspieces (78).

20. The air deodorizing device (10) as recited in any of claims 16 to 19, **characterized in that** the receiving sleeve (70) on its end facing the instrument panel (14) has a limit stop (80) for limiting the rotational motion of the cartridge housing (24).

21. A cartridge (16) for use in an air deodorizing device (10) as recited in any of the preceding claims, comprising a cylindrical cartridge housing (24) for accommodating a fragrance carrier, and outflow openings (22) that are arranged in the longitudinal direction of the cartridge housing (24), **characterized in that** the outflow openings (22) are arranged essentially over the entire length of the cartridge (16) and are separated from each other by crosspieces (26).

22. The cartridge (16) as recited in claim 21, **characterized in that** a free end of the cartridge housing (24) is closed by a cover (28).

23. The cartridge (16) as recited in claim 22, **characterized in that** the cover (28) can be removed without being destroyed.

24. The cartridge (16) as recited in claim 22 or 23, **characterized in that** the cartridge housing (24) at its end opposite the cover (28) has an integrally molded end section (34) which is firmly connected to a head part (38) that engages over the end section (34).

25. The cartridge (16) as recited in claim 24, **characterized in that** the end section (34) of the cartridge housing (24) has a cutout (36) in which a projection on the head part (38) engages, so that the head part (38) is clamped onto the end section (34).

26. The cartridge (16) as recited in claim 24 or 25, **characterized in that** the head part (38) has a cylindrical section (46) and a flange section (48) that protrudes radially from the cylindrical section (46).

27. The cartridge (16) as recited in claim 26, **characterized in that** the cylindrical section (46) at its end facing the cartridge housing (24) has at least one radially protruding attachment point (52) for fixing the cartridge (16) in position.

28. The cartridge (16) as recited in claim 27, **characterized in that** a marking (60) is arranged on a location of the flange section (48) that is opposite the attachment point (52).

29. The cartridge (16) as recited in any of claims 26 to 28, **characterized in that** the cylindrical section (46) at its end facing away from the cartridge housing (24) is provided with a cap (42).

30. The cartridge (16) as recited in any of claims 21 to 29, **characterized in that** the cartridge housing (24) includes sealing lips (62) that are arranged on the exterior side and run in the axial direction.

31. The cartridge (16) as recited in any of claims 21 to 30, **characterized in that** the cartridge (16) further includes a receiving sleeve (70) for the cartridge housing (24) having at least one through opening (76) arranged in the longitudinal direction, the cartridge housing (24) being rotatably arranged in the receiving sleeve (70) and being adapted to be moved from a closed position, in which the outflow openings (22) are completely covered by the receiving sleeve (70), to an open position, in which the through opening (76) is situated over the outflow openings (22).

32. The cartridge (16) as recited in claim 31, **characterized in that** the receiving sleeve (70) on its exterior circumference has at least one longitudinal rib (72) for fixing the receiving sleeve (70) in position.

33. The cartridge (16) as recited in claim 31 or 32, **characterized in that** the through opening (76) is subdivided by crosspieces (78).

34. The cartridge (16) as recited in any of claims 31 to 33, **characterized in that** the receiving sleeve (70) on one of its free ends has a limit stop (80) for limiting the rotational motion of the cartridge housing (24).

## Revendications

1. Dispositif de désodorisation d'air (10) pour l'utilisation dans des véhicules automobiles, comportant un boîtier (12) et une cartouche (16) insérée dans le boîtier (12) pour recevoir un support de parfum (79), le boîtier (12) étant agencé sur la face arrière d'un tableau de bord (14) du véhicule automobile et présentant au moins un orifice d'écoulement (18) qui est en communication fluidique avec des orifices d'aération (20) dans le tableau de bord (14), et la cartouche (16) présentant au moins un orifice d'échappement (22) correspondant à l'orifice d'écoulement (18) dans le boîtier (12), le dispositif de désodorisation d'air étant **caractérisé en ce que** la cartouche est agencée dans le boîtier (12) de telle sorte que la cartouche (16) soit mobile depuis une position fermée, dans laquelle l'orifice d'échappement (22) est entièrement recouvert, vers une position ouverte, dans laquelle l'orifice d'écoulement (18) se trouve au-dessus de l'orifice d'échappement (22).

2. Dispositif de désodorisation d'air (10) selon la revendication 1, **caractérisé en ce que** la cartouche (16) est agencée mobile en rotation dans le boîtier (12).

3. Dispositif de désodorisation d'air (10) selon la revendication 1 ou 2, **caractérisé en ce que** la cartouche (16) comprend un boîtier de cartouche (24) cylindrique.

4. Dispositif de désodorisation d'air (10) selon l'une des revendications 1 à 3, **caractérisé en ce que** la cartouche (16) présente des orifices d'échappement (22) radialement opposés et s'étendant en direction longitudinale de la cartouche (16).

5. Dispositif de désodorisation d'air (10) selon l'une des revendications 1 à 4, **caractérisé en ce que** les orifices d'échappement (22) sont agencés sensiblement sur toute la longueur de la cartouche (16) et sont séparés les uns des autres par des traverses (26).

6. Dispositif de désodorisation d'air (10) selon l'une des revendications 1 à 5, **caractérisé en ce que** l'extrémité de la cartouche (16) qui est détournée du tableau de bord (14) comporte un couvercle (28).

7. Dispositif de désodorisation d'air (10) selon la revendication 6, **caractérisé en ce que** le couvercle (28) peut être détaché sans être détruit.

8. Dispositif de désodorisation d'air (10) selon l'une des revendications 1 à 7, **caractérisé en ce que** la cartouche (16) présente un tronçon d'extrémité (34) qui est relié de manière solidaire à une partie de tête (38).

9. Dispositif de désodorisation d'air (10) selon la revendication 8, **caractérisé en ce que** le tronçon d'extrémité (34) de la cartouche (16) présente un évidement (36) dans lequel s'engage une saillie dans la partie de tête (38) de telle sorte que la partie de tête (38) est bloquée sur le tronçon d'extrémité (34).

10. Dispositif de désodorisation d'air (10) selon la revendication 8 ou 9, **caractérisé en ce que** la partie de tête (38) présente un tronçon cylindrique (46) et un tronçon de bride (48) faisant radialement saillie du tronçon cylindrique (46), et est guidée avec le tronçon cylindrique (46) à travers un orifice (50) dans le tableau de bord (14) de telle sorte que le tronçon de bride (48) est adjacent à la face avant du tableau de bord (14).

11. Dispositif de désodorisation d'air (10) selon la revendication 10, **caractérisé en ce que** le tronçon cylindrique (46) présente à son extrémité tournée vers la cartouche (16) au moins un point de fixation (52) qui fait saillie radialement et est poussé contre la face arrière du tableau de bord (16) par un élément ressort (56) agissant sur la cartouche (16).

12. Dispositif de désodorisation d'air (10) selon la revendication 11, **caractérisé en ce qu'**un repère (60) se trouve à un endroit du tronçon de bride (48), qui est opposé au point de fixation (52).

13. Dispositif de désodorisation d'air (10) selon l'une des revendications 10 à 12, **caractérisé en ce qu'**à son extrémité détournée de la cartouche (16), le tronçon cylindrique (46) est pourvu d'un capuchon (42).

14. Dispositif de désodorisation d'air (10) selon l'une des revendications 1 à 13, **caractérisé en ce que** le boîtier (12) comporte un orifice sur la face frontale qui correspond à un orifice (50) dans le tableau de bord (14) et dans lequel la cartouche (16) est insérée de manière à pouvoir être remplacée.

15. Dispositif de désodorisation d'air (10) selon l'une des revendications 1 à 14, **caractérisé en ce que** la cartouche (16) comporte des lèvres d'étanchéité (62) qui sont agencées sur la face extérieure et qui dans la position fermée rendent les orifices d'échappement (22) étanches par rapport à l'orifice d'écoulement (18) dans le boîtier (12).

16. Dispositif de désodorisation d'air (10) selon l'une des revendications 1 à 15, **caractérisé en ce que** la cartouche (16) présente un boîtier de cartouche (24) cylindrique et une douille de réception (70) pour le boîtier de cartouche (24), la douille de réception (70) étant agencée de manière à être immobile par rapport au boîtier (12), et le boîtier de cartouche (24) étant agencé mobile en rotation dans la douille de réception (70).

17. Dispositif de désodorisation d'air (10) selon la revendication 16, **caractérisé en ce que** sur sa périphérie extérieure, la douille de réception (70) présente au moins une nervure longitudinale (72) qui s'engage dans une rainure (74) correspondante dans le boîtier (12) et fixe la douille de réception (70) dans le boîtier (12).

18. Dispositif de désodorisation d'air (10) selon la revendication 16 ou 17, **caractérisé en ce que** la douille de réception (70) présente au moins un orifice de passage (76) qui est agencé en direction longitudinale et de manière à être en alignement avec l'orifice d'écoulement (18) dans le boîtier (12).

19. Dispositif de désodorisation d'air (10) selon la revendication 18, **caractérisé en ce que** l'orifice de passage (76) est divisé par des traverses (78).

20. Dispositif de désodorisation d'air (10) selon l'une des revendications 16 à 19, **caractérisé en ce qu'**à son extrémité tournée vers le tableau de bord (14), la douille de réception (70) présente une butée (80) pour limiter le mouvement rotatif du boîtier de cartouche (24).

21. Cartouche (16) pour l'utilisation dans un dispositif de désodorisation d'air (10) selon l'une des revendications précédentes, comportant un boîtier de cartouche (24) cylindrique pour la réception d'un support de parfum et des orifices d'échappement (22) agencés en direction longitudinale du boîtier de cartouche (24), **caractérisée en ce que** les orifices d'échappement (22) sont agencés sensiblement sur toute la longueur de la cartouche (16) et sont séparés les uns des autres par des traverses (26).

22. Cartouche (16) selon la revendication 21, **caractérisée en ce qu'**une extrémité libre du boîtier de cartouche (24) est obturée par un couvercle (28).

23. Cartouche (16) selon la revendication 22, **caractérisée en ce que** le couvercle (28) peut être détaché sans être détruit.

24. Cartouche (16) selon la revendication 22 ou 23, **caractérisée en ce que** le boîtier de cartouche (24) présente à son extrémité opposée au couvercle (28) un tronçon d'extrémité (34) moulé d'un seul tenant qui est relié de manière solidaire à une partie de tête (38) s'engageant sur le tronçon d'extrémité (34).

25. Cartouche (16) selon la revendication 24, **caractérisée en ce que** le tronçon d'extrémité (34) du boîtier de cartouche (24) présente un évidement (36) dans lequel s'engage une saillie dans la partie de tête (38) de telle sorte que la partie de tête (38) est bloquée sur le tronçon d'extrémité (34).

26. Cartouche (16) selon la revendication 24 ou 25, **caractérisée en ce que** la partie de tête (38) présente un tronçon cylindrique (46) et un tronçon de bride (48) faisant radialement saillie du tronçon cylindrique (46).

27. Cartouche (16) selon la revendication 26, **caractérisée en ce qu'**à son extrémité tournée vers le boîtier de cartouche (24), le tronçon cylindrique (46) présente au moins un point de fixation (52) faisant saillie radialement pour la fixation de la cartouche (16).

28. Cartouche (16) selon la revendication 27, **caractérisée en ce qu'**un repère (60) est agencé à un endroit du tronçon de bride (48), qui est opposé au point de fixation (52).

29. Cartouche (16) selon l'une des revendications 26 à 28, **caractérisée en ce qu'**à son extrémité détournée du boîtier de cartouche (24), le tronçon cylindrique (46) est pourvu d'un capuchon (42).

30. Cartouche (16) selon l'une des revendications 21 à 29, **caractérisée en ce que** le boîtier de cartouche (24) comporte des lèvres d'étanchéité (62) agencées sur la face extérieure et s'étendant en direction axiale.

31. Cartouche (16) selon l'une des revendications 21 à 30, **caractérisée en ce que** la cartouche (16) présente en outre une douille de réception (70) pour le boîtier de cartouche (24), comportant au moins un orifice de passage (76) agencé en direction longitudinale, le boîtier de cartouche (24) étant agencé mobile en rotation dans la douille de réception (70) et étant mobile depuis une position fermée, dans laquelle les orifices d'échappement (22) sont entièrement recouverts par la douille de réception (70), vers une position ouverte, dans laquelle l'orifice de passage (76) est situé au-dessus des orifices d'échappement (22).

32. Cartouche (16) selon la revendication 31, **caractérisée en ce que** sur sa périphérie extérieure, la douille de réception (70) présente au moins une nervure longitudinale (72) pour la fixation de la douille de réception (70).

33. Cartouche (16) selon la revendication 31 ou 32, **caractérisée en ce que** l'orifice de passage (76) est divisé par des traverses (78).

34. Cartouche (16) selon l'une des revendications 31 à 33, **caractérisée en ce qu'**à l'une de ses extrémités libres, la douille de réception (70) présente une butée (80) pour limiter le mouvement rotatif du boîtier de cartouche (24).
